(19) Europäisches Patentamt / European Patent Office / Office européen des brevets

(11) **EP 4 221 641 B1**

(12) **EUROPEAN PATENT SPECIFICATION**

(45) Date of publication and mention
of the grant of the patent:
**14.08.2024 Bulletin 2024/33**

(21) Application number: **21778222.6**

(22) Date of filing: **02.09.2021**

(51) International Patent Classification (IPC):
**A61F 2/70** (2006.01)     **A61F 2/72** (2006.01)
**A61F 2/76** (2006.01)

(52) Cooperative Patent Classification (CPC):
**A61F 2/72; A61F 2/70;** A61F 2002/704;
A61F 2002/762; A61F 2002/763; A61F 2002/764;
A61F 2002/7665

(86) International application number:
**PCT/IB2021/058004**

(87) International publication number:
**WO 2022/069969 (07.04.2022 Gazette 2022/14)**

(54) **PROSTHETIC DEVICE**

PROTHESENGERÄT

DISPOSITIF PROTHÉTIQUE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **30.09.2020 IT 202000023011**

(43) Date of publication of application:
**09.08.2023 Bulletin 2023/32**

(73) Proprietor: **BIONIT LABS SRL
73100 Lecce (IT)**

(72) Inventors:
• **MACRI', Chiara
73057 Taviano (LE) (IT)**
• **AVENTAGGIATO, Matteo
73024 Maglie (LE) (IT)**

(74) Representative: **Conversano, Gabriele
Laforgia, Bruni & Partners srl
Via Michele Garruba, 3
70122 Bari (IT)**

(56) References cited:
**WO-A2-2014/197401     US-A1- 2013 310 979
US-A1- 2016 278 947**

**Description**

The present invention relates to a myoelectric-controlled prosthetic device for calibration and usage of said device.

It is to specified that in the present document the term prosthetic device refers to a device comprising a prosthesis, an orthosis, an exoskeleton and relative control means.

In particular, the present invention relates to a myoelectric-controlled prosthetic device to be used with a particularly rapid and intuitive training of the patient, and not subject to physiological variations of the signal levels detected by the myoelectric sensors over the time.

Technical field

People born with a limb, and who, during their life, lose it in an accident or due to an illness, often keep the capacity to contract the muscles which articulated the limb previously. On the contrary, people born without a limb due to a genetic malformation or other neonatal illnesses or accidents have no feeling of "ghost limb" but can imagine anyway the movements the failing limb could carry out after determined muscle contractions thanks to the experience of the contralateral limb. There exists also a group of people who, owing to neuromuscular disorders, absence of stimulations from the environment or other physiological/environmental reasons, have not matured the expectation of a proprioceptive feedback of the muscular contraction effects on the amputated or missing limb.

US 2016/278947 is seen as the closest prior art document upon which the pre-characterising portion is based. The pre-characterising portion defines a myoelectric prosthetic device comprises:

- a limb or artificial joint or exoskeleton or orthosis comprising a mechanism provided with at least an actuator and configured to carry out one or more actions (for example a prosthetic hand can close and open, an exoskeleton arm can support the natural movement of the limb);
- a supplying source (for example an electric battery);
- at least an input source (and preferably more than one input source) modulated by the contraction of one or more muscles of the subject wearing the device (for example one or more electrodes for detecting surface muscular potentials, called surface electromyographic signals, sEMG);
- electronic computing means, on which computer programs are loaded, configured to detect the signals coming from said at least one input source and to actuate said at least one actuator in order that said device carries out an action as a function of the signals coming from said at least one input source.

According to embodiments known at the state of the art, the prostheses are directed to all the just described categories of amputees, with three possible approaches described in the following: in case of residual hypotrophic muscles, absence of space for positioning more than one electrode, antagonist muscles innervation loss, only one electrode is applied at one of the residual muscles used to activate the amputated or missing limb, and the prosthesis is actuated by carrying out two movements thanks to the setting of two thresholds on the amplitude of the sEMG signal measured for the unique electrode provided. For example, in case of prosthetic hand, this can open when the first threshold is reached, and close when the second threshold is reached, as it is described for example in Merletti at al Electromyography: Physiology, Engineering, and Non-Invasive Applications, 2004, IEEE Press Series on Biomedical Engineering.

In other cases, two electrodes are applied, one being positioned at agonist muscles and one at antagonist muscles. In this case, an actuation threshold is set for each electrode to control the movement of agonist/antagonist muscles. For example, in case of prosthetic hand the activation of the finger flexor muscles activates the hand closing and the activation of finger extensor muscles activates its opening).

Anyway, in this configuration it is needed to set a biunivocal correspondence between electrode and controlled movement. In the prosthetic hand example, the electrode positioned on the flexor muscles controls the closing movement while the electrode positioned on the extensor muscles controls the opening movement.

At the state of the art, for example in US10448857B2 and US9566016B2, prostheses are known that comprise a crown of three or more electrodes. In these systems, the electrodes have to be arranged with a precise order since the amplitude of the drawn sEMG signals can be used to pilot multi-parametric systems, such for example systems based on Kiviat diagrams whose axes represent the amplitude of each channel. In these cases, the subject is normally subjected to a difficult training step, during which he learns to carry out constantly the contractions needed for the generation of correct polygons of the Kiviat diagram which enable the prosthesis correct movements.

In the calibration of a prosthesis, it is also needed to calibrate the torque and/or the prosthesis activation speed. The most intuitive case is the one of a robotic hand, in which the activation torque determines the strength with which the hand grabs an object.

According to the calibration methods known at the state of the art, an EMG relation vs strength or muscle torque is imposed, possibly derived from the contemporaneous recording of the torque exerted by the hand joints of a healthy subject, and the electromyographic signals detected by the electrodes installed on the limb of such subject. Such relation between measured strength or torque and electromyographic signals is used to build a calibration curve used then for the prostheses worn by amputees. These relation curves EMG-strength or EMG-torque, when pre-determined by literature studies and applied directly on amputees, are little efficient since this kind of solution does not consider the subject muscle morphology and his anthropometric characteristics and, so, it is likely to not allow the subject to use the prosthesis instinctively and immediately.

Document US2016278947 describes a prosthetic system comprising computing means configured to acquire signals from an angular velocity sensor and to control the prosthesis as a function of these acquired signals.

Technical problem

All the devices known at the state of the art and in particular the methods that such devices use to associate the movements of the prosthetic device with the signals detected by the input sources are limited, since:

- if to activate the prosthesis movements thresholds on the sEMG signals amplitude are used, it is often needed to adapt them periodically upon physiological variations the signals detected over the time are interested by. In fact, it is known that weather variations and the physical activity carried out by the subject modify the skin microcirculation characteristics, the sweating and so the bioelectric characteristics of the electrode-skin coupling. The electro-skin impedance change leads to a change of the sEMG signal amplitude characteristics and so, the set thresholds are reached with higher or lower difficulty with the same muscle contraction carried out. So, this requires periodical interventions by the orthopedic technician for adjusting the threshold values,
- if to activate the prosthesis movement, pattern recognition techniques are used by means of supervised machine learning procedures, the settings are to be modified over the time owing to the electrode-skin contact impedance variations or owing to muscle tissues physiological variations causing intensity variations of the detected signals. In fact, the usage of myoelectric prostheses leads the amputee or the subject born with a malformation to contract more often the muscles used to pilot the prosthesis. These contractions act as a sort of muscle training which manifests itself both as an increase in tissue volume and as more ability and capacity of the subject to contract such muscles selectively.

Another problem is to calibrate the torque and/or activation speed of a prosthesis or an orthosis worn by amputees who have only residual muscles or were even born without any limbs. In these subjects the calibration has to be carried out without any kind of feedback and by subjects who have never known, in their life, the feeling of moving the limb substituted by the prosthesis.

Generally, it is needed to guarantee stability to the movement recognition algorithm. Stability means the capacity of an algorithm to recognize the instruction expressed by the subject with low error, without often ending in error situations which could lead to the impossibility to manage/pilot the prosthesis. This is a condition potentially developable by all the systems integrating machine learning algorithms (such for example fuzzy decisional algorithms or neuronal nets, in particular not linear ones). These results are not reached by the calibration methods known at the state of the art.

Aim of the invention

So, the present invention provides a prosthetic device which overcomes the limits linked to the embodiments known at the state of the art, and in particular which eliminates the need of configuration for the user or technician, which does not require the association of each electrode with a specific movement, which can be used indifferently with any number of electrodes since the number of connected electrodes does not increase the configuration or calibration complexity.

Yet, the present invention provides a prosthetic device able to adapt autonomously and automatically its own functioning to the final user physiologic characteristics, and which is able to adapt its own configuration parameters automatically over the time, so that a correct functioning is kept even while the user physiological conditions vary or while the environmental conditions in which the device is used vary, without any intervention of the technician or final user.

The present invention relates to a prosthetic device as set out in the claims.

The device comprises also preferably:

- at least a sensor, and preferably a plurality of sensors, integrated in the system and configured to detect information about the status of the device or its components. For example, there can be provided one or more temperature sensors, one or more inertial units for detecting the relative position in the space of the various components of the device, one or more pressure sensors for detecting the contact with outer objects;

- a mechanic protection against the outer environment (for example a cosmetic glove in impermeable and/or shockproof material, in case of a transradial prosthesis).

The prosthetic device according to the invention is characterized in that, on said electronic computing means computer programs are loaded configured to carry out the method described in the following. The method according to the invention comprises the steps of:

(0) checking the presence of calibration parameters in the memory;

- in affirmative case, carrying out a calibration test procedure (2), in negative case, carrying out a supervised calibration procedure (1), after which carrying out the calibration test procedure (2);
- at the result of the calibration test procedure (2), in positive case going to the device using condition (3) and not supervised calibration (4), in negative case repeating the supervised calibration.

In order to carry out the supervised calibration procedure (1), the method comprises the next steps. (100) recording the signals (Ns) relative to each electrode provided in the system with the user in a predetermined condition (rest condition or condition of execution of a predetermined movement) for a predetermined time interval $\Delta T$, thus obtaining N tracks or - pieces - of electric signal in the time domain, one relative to each sensor.

It is to be specified that rest condition means a condition in which the subject is asked to remain still, with relaxed muscles, in an inactivity state, thus avoiding contracting muscles, avoiding exerting pressure on the portions of the prosthetic device and avoiding activating possible sensors provided in the system. Said predetermined time interval, according to the kind of connected electrodes and the kind of prosthetic actuator, can be varied, for example, between 1 and 10 seconds.

(110) subdividing each of said signals (Ns) in a plurality of time intervals (also called epochs or windows) of predetermined duration, thus obtaining "M" signal pieces for each signal. The duration of each window, according to the kind of signals and the kind of prosthetic actuator, is preferably between 200 ms and 2 seconds. It is also to be specified that the time windows can be partially overlapping or not. The overlapping allows to have a greater number of windows at the same signal duration, thus maximizing the extraction of information contained therein, also in presence of noise; (120) for each one of the M windows of each recorded signal, extracting at least a characteristic parameter (feature) relative to the signal in the time domain, or relative to the signal FFT transform. The extracted features comprise preferably one or more of the following ones: peak or peak-to-peak average amplitude, peak or peak-to-peak maximum amplitude, effective value, power spectrum average frequency, power spectrum median frequency, -6dB power spectrum band, -20dB power spectrum band. In this way, it is obtained a matrix $F = K \times M$, wherein K is the number of computed features, M is the number of epochs and each element $F(i,j)$ of the matrix is the value of the $i^{th}$ feature calculated on the $j^{th}$ signal window; (130) calculating a statistical estimator of the value of each feature calculated at point (120). In a preferred embodiment, said statistical estimator is the median M; in another embodiment said statistical estimator is the signal average. In this way, it is obtained a vector of dimensions $K \times 1$, wherein K is the number of feature considered.

The vector defines the coordinates of a point representing the rest condition (rest) or the predetermined movement of point (100) in a K-dimensional space, each dimension being relative to a signal feature.

The above-described procedure can comprise one or more of the following variations:

Possibly at point (110), both in case of acquisition of the values relative to the rest position and in case of acquisition of the values relative to a predetermined movement, the starting portions of the recorded signals (for example a portion between 5 and 20%, according to the recording conditions) can be discarded to be certain that the subject is really in the condition of rest or of execution of the predetermined movement, since in the starting step the subject cannot be in the required condition yet.

Possibly after point (130), the point can follow of (131) calculating, for each feature, the standard deviation of the values distribution, comparing each feature with its own statistical estimator, and discarding the values with a difference - in absolute value - greater than twice the standard deviation from said statistical estimator.

Concerning the acquisition of signals relative to a predetermined movement, it is to be specified that the kind of muscle contraction to be associated with the movement actuated by the mechanism of the device can be different from the kind of contraction a not amputee would carry out for carrying out the same movement.

For example, a transhumeral amputee has not the forearm muscles that he would normally use to activate the finger flexing, but he could use the flexor muscles of the forearm (one or two heads of the biceps brachii). Similarly, a trans-radial amputee, even if he has still the muscles used for the natural flexion of the fingers, in order to optimize the piloting of the movement relative to the finger flexion could prefer to carry out the muscular contraction associated with the wrist flexion, since the muscular group contracting in this case is mainly concentrated on the inner side of the forearm, thus exposing the detecting electrodes to a lower cross-talk phenomenon.

Similarly, in case of opposite movement (in the example the finger extension), a trans-humeral amputee has not the forearm muscles that would normally use to activate the finger extension, but he can use the extensor muscles of the forearm (one or more heads of the triceps brachii). Similarly, a trans-radial amputee, even if he has still the muscles used for the natural finger extension, in order to optimize the piloting of the movement relative to the finger flexion can prefer to carry out the muscular contraction associated with the wrist extension, since the muscular group contracting in this case is mainly concentrated on the outer side of the forearm, thus exposing to a lower cross-talk phenomenon.

The steps (100) to (131) are carried out sequentially for a rest condition (rest) and for a plurality of predetermined movements (Mov_1, Mov_2,..., Mov_n), thus obtaining a plurality of vectors of dimensions K x 1 defining each, in the K-dimensional space relative to the K acquired features, the position of a point representing each detected movement.

The method provides preferably the execution of the above steps sequentially for a movement and in the following for the movement opposite thereto. For example, in case of trans-radial prosthesis, if the first calibrated movement (Mov_1) is the finger flexion, the second movement (Mov_2) is normally the finger extension.

Moreover, preferably, at the end of the calibration procedure for all the movements intended, the calibration of the rest condition (rest) is carried out again in order to compensate possible alterations of the sEMG signal features upon the introduction of muscular fatigue phenomena. In order to do so, the steps (100) to (131) for the rest condition are carried out, thus obtaining the definition of another point representing the rest condition in K-dimensional space. The point representing the rest condition is then calculated as the average point between the first and the second point calculated for said condition.

The rest condition can be also object of detection before and after the detection relative to each movement, by calculating for each of the K features relative to the rest condition the average value of all the values calculated for each detection, in order to define the point representing the rest condition in the K-dimensional space.

According to a preferred embodiment, the prosthetic device according to the invention comprises at least a position sensor, configured to detect the position of said device, and is configured to store for each movement, as well as for the rest condition, a plurality of reference points and to select automatically the suitable reference point to be considered during usage as a function of the position of said prosthetic device detected by said position sensor. Said position sensor is preferably an inertial unit (IMU, inertial measuring unit).

This characteristic satisfies the need to compensate possible alterations of the signals recorded by the sensors due to the position variation of the prosthetic device. For example, in case of a trans-radial prosthesis piloted by one of more EMG sensors, the variation of the arm position can cause the variation of the relative position electrode-muscle even in absence of variation of the relative position electrode-skin. In this case, by using an IMU positioned in the storage (integrated or not in the electrodes, or in the storage itself or in the battery housing, or in other components of the prosthesis), it is possible to recognize the position of the prosthesis and, on the basis thereof, to select automatically and to use the most suitable calibration parameters.

In order to calibrate the functioning of the device in a plurality of positions, the supervised calibration procedure (1) comprises the steps of:

(80) defining a plurality of significant positions $(P_1,..., P_r)$ ;
(85) making the user wearing the device assume the first of said positions (P1);
(90) carrying out the steps (100) to (131), while storing a first set of reference points relative to each movement carried out with the device in said first position (P1);
(95) repeating the steps (85) and (90) for all the positions $(P_1,..., P_r)$ defined at point (80) while storing for each one thereof a relative set of reference points relative to each movement carried out with the device in said position.

After the execution of the supervised calibration procedure, in order to increase the calibration stability (i.e. in order to reduce the error frequency), the method can provide the execution of the following steps:
(140) calculating the distance in the K-dimensional space between the point representing each kind of movement or rest and all the other points representing further conditions of movement or rest.

It is to be specified that in a first embodiment the distance of point (140) can be calculated as Euclidean distance in a K-dimensional space; in a second embodiment at each dimension of K-dimensional space a specific "weight" can be assigned, and the distance can be calculated as the Euclidean distance in the K-dimensional space in which each addend of the summation of the squares of the distances along each axis is multiplied by its own weight.

(150) comparing said distances calculated at point (140) with a minimum threshold of predetermined distance;
(160) in case of presence of points representing the movements distant from the points representing the other movements less than the threshold of point (150), individuating said movements and sending a warning message to the user;
(170) as a function of a user decision, carrying out again the supervised calibration procedure or eliminating one or more movements selected by the user among the ones individuated at point (160).

In this way, it is obtained the definition of a series of points representing a plurality of movements distant to each other more than the minimum threshold. This reduces the possibility of error in the next step of device usage, as it will be clearer in the following.

Once the supervised calibration step according to the just described procedure is ended, the method provides the possibility to carry out a test (2) of the recorded parameters, according to the following steps:

(200) detecting the parameters relative to the signals of each electrode while the user carries out one specific movement,
(210) calculating, with the same modes described in steps (110) to (130), the coordinates of the point representing the movement carried out by the user.
(220) individuating, among all the points representing the movements (or the rest condition) stored during the supervised calibration procedure, the one with the lower distance from the point defined at step (210).
(230) providing the user with a feedback indicating the movement individuated at point (220).

It is to be specified that in a first embodiment, the distance of point (220) can be calculated as Euclidean distance in a K-dimensional space; in a second embodiment at each dimension of the K-dimensional space a specific "weight" can be assigned, and the distance can be calculated as the Euclidean distance in K-dimensional space in which each addend of the summation of the squares of the distances along each axis is multiplied by its own weight.

So, the user can consider the test procedure as successful in case the feedback of point (230) indicates the movement he was really carrying out. In case the recognition of movements is not satisfying, the user can repeat the supervised calibration procedure (1), by repeating it as a whole or only for some movements (or for the "rest" condition). In case, instead, the configuration obtained with the supervised calibration is satisfying, it is validated and used for the next steps.

Clearly, in case a plurality of sets of reference points relative to a plurality of significant positions of the device are provided, the test procedure (2) can be conveniently carried out for each of said significant positions.

It is to be specified that the feedback provided at step (230) - as also the warning of step (160) - can be:

a. a visual feedback of text kind through a user interface showing the recognized movement with a message ("rest", "movement 1: hand closing", "movement 2: hand opening", etc.).
b. a visual feedback on a software user interface connected to the system which, through the virtual representation in 2D or 3D of the prosthesis or orthosis or exoskeleton used, shows in real time in animated or static way how the actuated movement would be.
c. visual feedback of a dynamic histogram, where each bar relates to one of the features analyzed on the inputs and it varies its own value as a function of the measured intensity;
d. acoustic feedback where each feature analyzed on the inputs modulates a "note" through the modulation both in amplitude and frequency of a carrier frequency as a function of the value assumed by the relative feature;
e. visual feedback provided by the same prothesis or orthosis or exoskeleton by means of the actuation of the real movement intended.
f. haptic feedback.

Moreover, preferably, at step (230), the device is configured to provide the user with an indication relative to the distance between the point calculated at step (210) and the point individuated at step (220). The indication can be provided by means of various feedback tools:

a. visual feedback of text kind through a graphic interface showing a numeric indication (percentage or absolute) of the correspondence between the instruction expressed and the movement selected as target;
b. visual feedback on a software user interface connected to the system which, through the virtual representation in 2D or 3D of the prosthesis or orthosis or exoskeleton used, shows in real time the effective recognition of the movement selected thorough animated or static images;
c. visual feedback of a dynamic histogram, where each bar relates to one of the features analyzed on the inputs and it varies its own value as a function of the measured intensity; in particular, it is provided a reference (for example a line or other type of marker on the same bar of the histogram) highlighting the target intensity level for each of the analyzed features;
d. acoustic feedback where each feature analyzed on the inputs modulates a "note" through the modulation both in amplitude and in frequency of a carrier frequency as a function of the value assumed by the relative feature; in order to provide another feedback, the amplitude of the sounds can increase while approaching the target combination for the selected movement;
e. visual feedback provided by the same prothesis or orthosis or exoskeleton by means of the actuation of the same movement selected.

After the calibration test procedure (2), in positive case, the method provides to carry out the procedure of usage of the device (3) and the not supervised calibration (4).

The procedure of usage of the device (3) provides the steps of:
(300) acquiring, at predetermined time intervals, the signals (Ns) coming from said electrodes for a predetermined duration and storing them in a relative buffer.

Said predetermined time intervals are preferably between 200 ms and 1000 ms; said predetermined duration is preferably between 200 ms and 2000 ms.

(310) subdividing each of said signals (Ns) in a plurality of time intervals (also called epochs or windows) of predetermined duration, thus obtaining "M" signal pieces for each signal. The duration of each window, according to the kind of signals and the kind of prosthetic actuator, is preferably between 200 ms and 2 seconds. It is also to be specified that the time windows can be partially overlapping or not. The overlapping allows to have a greater number of windows at the same signal duration, thus maximizing the extraction of information contained therein, also in presence of noise.

(320) for each one of the M windows of each recorded signal, extracting at least a characteristic parameter (feature) relative to the signal in the time domain, or relative to the signal FFT transform. The features extracted during the using step are the same features extracted in the calibration step at step (120),

(330) calculating a statistical estimator of the value of each feature calculated at point (320). The calculated statistical estimator has to be the same used in the calibration step at point (130). In this way, it is obtained a vector of dimensions K x 1, wherein K is the number of features considered.

The calculation modes adopted, including the discarding of the values distant from the statistical descriptor, are the same used in the supervised calibration step.

(340) calculating the Euclidean distance between the point calculated at step (330) and each point representing each movement stored during the supervised calibration procedure, thus identifying the movement for which such distance is minimum.

(350) carrying out the movement identified at step (340) .

It is clear that, generally, the word "movement" can mean also the "rest" condition.

Moreover, in case a plurality of sets of reference points relative to a plurality of significant positions (P1, ..., Pr) are provided, the procedure of usage of the prosthetic device (3) comprises, before the step (300), the steps of:

(298) acquiring by means of said position sensor the position of the device, thus identifying the significant position of reference;

(299) selecting the set of reference points representing each movement relative to the position identified at point (298). The above-described procedure can comprise one or more of the following variants:

- the values of the medians can be substituted with the average values or of other statistical estimator according to the kind of feature considered;
- the starting portions of the recorded signals (for example a portion between 5 and 20%, according to the recording condition) can be discarded to be certain that the subject is really able to express the instruction of possible movement in a stable condition.

Moreover, in order to reduce the errors of rest condition recognition, defined as $D_{MOV}$ the distance of the point individuated at step (330) from the point representing the movement identified at step

(340) and as $D_{REST}$ the Euclidean distance of the point individuated at step (330) from the point representing the rest condition, at step (350) the following condition can be added:

- if $D_{MOV} \leq \alpha \cdot D_{REST}$ carrying out the movement identified at step (340),
- if $D_{MOV} > \alpha \cdot D_{REST}$ keeping the device in rest condition.

Wherein $\alpha$ is a suitable coefficient equal or lower than 1 and greater than 0.

In particular, the more $\alpha$ is low, the lower the system is sensible to the actuation of movements; for too low values of $\alpha$ the system could not recognize any movement. Alfa is preferably between 0.75 and 0.95.

It is now described the procedure "not supervised calibration" (4).

As yet said, this procedure allows to consider automatically and in absolutely transparent manner for the user, all the variations over the time due to factors such as: variations of the impedance of the electrode-skin contact, physiological variations of the muscle tissues due to fatigue, muscle training or vascularization change, alterations of signals recorded by sensors sensible to environmental conditions.

More specifically, this not supervised calibration procedure (4) intervenes automatically to modify, if needed, the reference points in the multidimensional space relative both to the rest condition and to each movement.

The procedure provides the following steps:
(400) with the prosthetic device used, starting a timer (countdown) of predetermined period.
Said period is preferably between 60 seconds and 30 minutes.
(410) storing the values of the features relative to the first movement following the timer expiring of point (400) associated with the rest condition, thus defining the point Rest_1 in the K-dimensional space, and starting a new timer of predetermined period.
(420) storing the values of the features relative to the first movement following the timer expiring of point (410) associated with the rest condition, thus defining the point Rest_2 in the K-dimensional space.
(430) calculating the Euclidean distances of the two points defined at steps (410) and (420) from the reference point representing the rest condition (Rest_ref).
(440) In case the distance from the reference point (Rest_ref) of the point (Rest_2) defined at step
(420) is greater than the distance from the reference point (Rest_ref) of point (Rest_1) defined at step (410), updating the values of the reference point, on the contrary, not updating said values.
(450) in case at step (440) it is needed to update the values, calculating the value of each $i^{th}$ feature of the new reference point relative to the rest condition (Rext_ref') as

—

$$Rest\_ref'(i) = Rest\_ref(i) + b \cdot [Rest\_2(i)] - Rest\_ref(i)]$$

Wherein b is a coefficient of value between 0 and 1, and preferably between 0.05 and 0.2.
(460) substituting the values of the features relative to the first movement associated with the rest condition individuated at point (410) with the values of the second movement associated with the rest condition individuated at point (420), and starting from point (420) again.
The timer period at point (400) can be modified manually by the user or automatically by the system, in order to compensate possible factors which could alter the features measured during the step of "usage of the prosthetic device", as for example the variation of the electrode-skin impedance measured directly (through a suitable impedance sensor) or indirectly (through the measure of environmental parameters as temperature and humidity).
The modification of the timer period can be actuated in response to:

    i. variations of the value detected by one or more sensors of the system (such as a sensor of the electrode-skin impedance, a sensor of skin temperature or humidity, a sensor for measuring the environmental temperature or humidity, etc.);
    ii. variations of the intensity of the activity carried out by the user detected for example by an inertial unit;
    iii. after the manual modification provided by the user through a suitable interface with the system (as for example a potentiometer, an ON/OFF button, a toggle button, a graphic interface on dedicated software, etc.);

It is described now the calibration method for controlling the sensors requiring a proportional control of the torque and/or speed, actuated by the prosthesis or orthosis or exoskeleton.
For example, in case of a transradial prosthesis, it is possible to associate the control of the torque and/o speed with the finger closing so that it is proportional to the muscular contraction exerted by the subject.
So, for the proportional control it is needed to derive a command value, to be used for the proportional control of the actuator, from the signals detected by the sensors (electromyography sensors, MMG sensors (mechanomyogram), AMG sensors (acoustic myogram)).
Also in this case, as for the calibration procedure, the procedure can be repeated separately for a plurality of different positions of usage.
It is to be specified that the calibration procedure to manage the proportional control of an actuator is carried out conveniently after the just described supervised calibration procedure, and so, the records of the signals detected in the time domain are present in the memory, and in particular the signals recorded by the sensors at the execution of the movement are present in the memory for which the proportional control is to be calibrated. The method comprises the steps of:
(1000) defining the mathematical form of a function of dependency of the output value of the proportional control as a function of the features calculated for the signal recorded by each sensor, wherein said output value is normalized in values 0 to 1 (or analogously 0% to 100%).
Said function contains the summation of at least a term directly proportional to each $i^{th}$ feature by means of a respective coefficient ($\omega 1_i$). Preferably, said function contains also the summation of at least a term depending exponentially on the value calculated for each $i^{th}$ feature by means of a respective coefficient ($\omega e_i$). According to an embodiment said mathematical form can contain at least a term directly proportional to the $n^{th}$ power of the value calculated for the

$i^{th}$ feature, by means of a respective coefficient ($\omega n_i$).

In other terms, the function used is a function of the following kind, in which there are zero, one or more summations next to the first one.

$$V\% = \sum_{i=1}^{K} \omega 1_i \cdot Fi + \sum_{i=1}^{K} \omega n_i \cdot Fi^n + \sum_{i=1}^{K} Fi \cdot e^{\omega n_i}$$

(1100) setting with value equal to one the values of all the coefficients ($\omega e_i$, $\omega n_i$) which are not relative to the direct proportionality;

(1200) estimating the values of said coefficients of direct proportionality ($\omega 1_i$) by means of an error reduction algorithm which for a plurality of percentage values of the instruction to be used for the actuator imposes that the summation of the maximums recorded during the execution of the movement for each feature ($Fi\_max$), multiplied each by the respective coefficient of proportionality ($\omega 1_i$) and by the considered percentage value, is equal to the percentage values of the instruction to be used for the actuator.

Substantially a plurality of relations as the following one are written, each one for a different value of instruction for the actuator

$$V\% = \sum_{i=1}^{K} \omega 1_i \cdot V\% \cdot Fi\_max$$

The relations are then used to estimate, according to technique known per se at the state of the art, the values of the coefficients of direct proportionality ($\omega 1_i$) which reduce error.

It is to be noted that, for the estimation of the coefficient values, no measured torque value has been used, but only the values deriving from EMG sensors.

(1300) making the user repeat the movement object of the calibration, asking him to increase gradually the torque applied, and providing him with a feedback by means of graphic interface of the applied torque, calculated with the coefficient values estimated at point (1200) and the relation defined at point (1000), in which only the terms relative to the direct proportionality are valued.

(1400) calculating the features of the signals detected by the EMG sensors during step (1300) and estimating the coefficient values of the relation defined at point (1000) according to what described at point (1200) but considering also the exponential terms and/or the ones with proportionality higher than the first one.

(1500) repeating the steps (1300) and (1400) up to when the estimated error goes under a predetermined threshold.

It is to be noted that also in this case no measured torque value has been used, but only the values of EMG signals.

The feedback of point (1300) can be matched and/o substituted with various kinds of proportional feedbacks, as for example:

- vibratory/haptic feedback: during the execution of contraction ramps a vibration proportional (in frequency and/or amplitude) to the intensity of the contraction to be developed is exerted through a vibratory motor or a system of vibratory motors;
- acoustic feedback: during the execution of contraction ramps it is provided an acoustic signal proportionally modulated in amplitude and/or frequency to the intensity of the muscle contraction to be developed;
- visual feedback: further and/or alternative visual feedbacks, as for example:
- LED, LED bars or other lighting systems to provide in output a proportional light signal in colour and/or light intensity proportionally modulated to the intensity of the muscle contraction to be developed;
- text indications on the same display/monitor used for the calibration steps and/or on an accessory display/monitor so that text indications are provided (through words, absolute or percentage numbers) proportionally modulated to the intensity of the muscle contraction to be developed.

## Claims

1. Prosthetic device comprising:

   - a limb or artificial joint or orthosis or an exoskeleton comprising a mechanism provided with at least an actuator and configured to carry out one or more actions;
   - a power supplying source;

- at least an input source modulated by the contraction of one or more muscles of the subject wearing the device, comprising at least an electrode;
- electronic computing means, on which computer programs are loaded, configured to detect the signals coming from said at least one input source and to actuate said at least one actuator in order that said mechanism carries out an action as a function of the signals coming from said at least one input source,

**characterized in that**

on said computing means computer programs are loaded configured to carry out the method comprising the steps of:

(100) recording the signals (Ns) detected by said at least one electrode while said subject wearing the device is in a predetermined condition of movement or rest, for a predetermined time interval $\Delta T$;

(110) subdividing each of said signals (Ns) in a plurality of time intervals of predetermined duration;

(120) extracting from the signal relative to each of the time intervals defined at step (110) at least a characteristic parameter (feature) relative to the signal selected from the list comprising: peak or peak-to-peak average amplitude, peak or peak-to-peak maximum amplitude, effective value, power spectrum average frequency, power spectrum median frequency, -6dB power spectrum band, -20dB power spectrum band;

(130) calculating a statistical estimator for each feature calculated at step (120),

(140) repeating the steps (100) to (130) sequentially with said subject in rest condition and, in the following, with said subject carrying out a known movement selected from a list of a plurality of predetermined movements (Mov_1, Mov_2,..., Move_n) for each iteration,

(150) associating a vector defined by the set of the values of the features calculated at step (130) with each movement known carried out by the subject and with the rest condition of step (140), and storing said vectors.

2. Device according to claim 1, **characterized in that** said method comprises also a test procedure, comprising the steps of:

(200) detecting the signals detected by said at least one electrode while the user carries out one specific movement,

(210) calculating, with the same modes described in steps (110) to (130), a plurality of statistical estimators, and defining a vector defined by the set of the values of said statistical estimators;

(220) individuating, among all the vectors stored at step (150) and associated with a different movement, the one with the lower Euclidean distance from the point defined at step (210);

(230) providing the user with a feedback indicating the movement individuated at step (220) .

3. Device according to claim 2, **characterized in that** said method provides at step (230) to provide also the user with an indication relative to the Euclidean distance between the vector calculated at step (210) and the vector individuated at step (220) .

4. Device according to any one of the preceding claims, **characterized in that** said method provides, after step (230), a procedure of usage of said device, comprising the steps of:

(300) acquiring, at predetermined time intervals, the signals (Ns) coming from said at least one electrode, for a predetermined duration and storing them in a relative buffer.

(310) subdividing each of said signals (Ns) in a plurality of time intervals of predetermined duration, thus obtaining "M" signal pieces for each signal.

(320) for each one of the signal piece extracted at step (310) extracting at least a characteristic parameter (feature) according to the same modes followed at step (120).

(330) calculating a statistical estimator of the value of each feature calculated at step (320) according to the same modes followed at step (130) and defining a vector defined by the set of the values of said statistical estimators;

(340) calculating the Euclidean distance between the vector calculated at step (330) and each vector stored at step (150) and associated with a specific movement, thus identifying the movement for which such distance is minimum.

(350) carrying out the movement identified at step (340).

5. Device according to claim 4, further comprising a position sensor configured to recognize a plurality of positions of reference and **characterized in that** said method provides to repeat the steps (100) to (150) for each one of said positions of reference, storing for each one and for each movement of step (140) as well as for the rest condition a vector defined by the set of the values of the features calculated at step (130) .

6. Device according to claim 5, **characterized in that** said method comprises also, before step (300), the steps of:

(298) acquiring by means of said position sensor the position of the prosthetic device, thus identifying the significant position of reference;
(299) selecting the set of reference positions representing each movement relative to the position identified at step (298).

7. Device according to any one of the preceding claims, **characterized in that** said method comprises also an automatic updating procedure of the vectors stored at step (150), comprising the steps of:

(400) with the device being used, starting a timer of predetermined period;
(410) storing the values of the features relative to the first movement following the timer expiring of step (400) associated with the rest condition, thus defining a first vector (Rest_1) associated with said first movement associated with the rest condition, and starting a new timer of predetermined period;
(420) storing the values of the features relative to the first movement following the timer expiring of step (410) associated with the rest condition, thus defining a second vector (Rest_2) associated with the rest condition;
(430) calculating the Euclidean distances of each one the two vectors defined at steps (410) and (420) from the reference vector representing the rest condition (Rest_ref) stored at step (150) ;
(440) in case the distance between the reference vector (Rest_ref) stored at step (150) and said second vector associated with the rest condition defined at step (420) is greater than the distance between the reference vector (Rest_ref) stored at step (150) and said first vector associated with the rest condition defined at step (410), updating the values of said reference vector according to what known at step (450), on the contrary, not updating said values.
(450) in case at step (440) it is needed to update the values, calculating the value of each ith feature of the new reference vector relative to the rest condition (Rext_ref') as Rest_ref'(i) = Rest_ref(i) + b · [Rest_2(i)] - Rest_ref(i)] wherein b is a coefficient of value between 0 and 1,
(460) substituting the values of the features relative to the first movement associated with the rest condition individuated at step (410) with the values of the second movement associated with the rest condition individuated at step (420), and starting from step (420) again.

8. Device according to any one of the preceding claims, comprising at least an actuator provided with proportional control of the torque and/or speed actuated by the prosthesis or orthosis or exoskeleton, and **characterized in that** said method comprises the steps of:

(1000) defining the mathematical form of a function of dependency of the output value of said proportional control as a function of the features calculated for the signal recorded by each sensor, said mathematical form comprising the summation of at least a term directly proportional to each ith feature by means of a respective coefficient ($\omega1i$),
(1100) setting with a value equal to 1 the values of all the coefficients ($\omega ei$, $\omega ni$) defining said mathematical form and which are not relative to the direct proportionality to a feature;
(1200) estimating the values of said coefficients of direct proportionality ($\omega1i$) defined at step (1000) by means of an error reduction algorithm which, for a plurality of percentage values of the instruction to be used for the actuator, imposes that the summation of the maximums recorded during the execution of the movement for each feature (Fi_max), multiplied each by the respective coefficient of proportionality ($\omega1i$) and by the considered percentage value, is equal to the percentage values of the instruction to be used for the actuator.
(1300) making the user repeat the movement object of the calibration, asking him to increase gradually the torque applied, and providing him with a feedback by means of graphic interface of the applied torque, wherein said applied torque is calculated by means of said function defined at step (100), with the values of the coefficients estimated at step (1200) .

**Patentansprüche**

1. Prothetische Vorrichtung, umfassend:

   - ein Glied oder künstliches Gelenk oder eine Orthese oder ein Exoskelett, das einen Mechanismus umfasst, der mit mindestens einem Aktuator ausgestattet und so konfiguriert ist, dass er eine oder mehrere Aktionen ausführt;
   - eine elektrische Stromquelle;
   - mindestens eine Eingabequelle, die durch die Kontraktion eines oder mehrerer Muskeln des Subjekts, die Vorrichtung trägt, moduliert wird und mindestens eine Elektrode aufweist;
   - elektronische Berechnungsmittel, auf denen Computerprogramme geladen sind, die so konfiguriert sind, dass sie die von der mindestens einen Eingabequelle kommenden Signale erkennt und den mindestens einen Aktuator betätigt, so dass der Mechanismus eine Aktion in Abhängigkeit von den von dieser mindestens einen Eingabequelle kommenden Signalen ausführt,

   **dadurch gekennzeichnet, dass**
   Computerproramme auf den Computermittel geladen sind, die so konfiguriert sind, dass sie das Verfahren ausführen, das die folgenden Schritte umfasst:

   (100) Aufzeichnen der von mindestens einen Elektrode erfassten Signale (Ns) während eines vorbestimmten Zeitintervalls ∆T, während sich das die Vorrichtung tragende Subjekt in einem vorbestimmten Bewegungs- oder Ruhezustand befindet;
   (110) Unterteilen jedes der Signale (Ns) in mehrere Zeitintervalle vorgegebener Dauer;
   (120) Extrahieren aus dem Signal, das sich auf jedes der in Schritt (120) definierten Zeitintervalle bezieht, mindestens einen charakteristischen Parameter (Eigenschaft), der sich auf das aus der Liste ausgewählte Signal bezieht, umfassend: durchschnittliche Spitze oder Spitze-zu-Spitze-Amplitude, maximale Spitze oder Spitze-zu-Spitze-Amplitude, tatsächlicher Wert, mittlere Frequenz des Leistungsspektrums, mediane Frequenz des Leistungsspektrums, -6-dB-Leistungsspektralband, -20-dB-Leistungsspektralband;
   (130) Berechnen eines statistischen Schätzers für jedes in Schritt (120) berechnete Merkmal,
   (140) Wiederholen der Schritte (100) bis (130) nacheinander, während sich das Subjekt in Ruhe befindet und anschließend das Subjekt eine bekannte Bewegung ausführt, die jeweils aus einer Liste einer Vielzahl vorgegebener Bewegungen (Mov_1, Mov_2, ..., Move_n) für jede Iteration ausgewählt wird,
   (150) Verknüpfen eines Vektors, der durch die Anzahl der Werte der in Schritt (130) berechneten Merkmale, mit jeder bekannten Bewegung des Subjekts und mit der Ruhebedingung von Schritt (140), definiert ist, und Speichern dieser Vektoren.

2. Vorrichtung nach Anspruch 1, **dadurch gekennzeichnet, dass** das Verfahren auch ein Testverfahren umfasst, die Schritte umfassend:

   (200) Erfassen der von der mindestens einen Elektrode erfassten Signale, während der Benutzer eine bestimmte Bewegung ausführt,
   (210) Berechnen mehrerer statistischer Schätzer mit denselben in den Schritten (110) bis (130) beschriebenen Modi und Definieren eines Vektors, der durch alle Werte der statistischen Schätzer definiert ist;
   (220) Individualisieren, unter allen in Schritt (150) gespeicherten und einer anderen Bewegung zugeordneten Vektoren denjenigen, der den geringeren euklidischen Abstand von dem in Schritt (210) definierten Punkt aufweist;
   (230) Bereitstellen einer Rückmeldung an den Benutzer, die die individualisierte Bewegung in Schritt (220) angibt.

3. Vorrichtung nach Anspruch 2, **dadurch gekennzeichnet, dass** das Verfahren in Schritt (230) vorsieht, dem Benutzer auch eine Angabe bezüglich des euklidischen Abstands zwischen dem in Schritt (210) berechneten Vektor und dem in Schritt (220) individualisierten Vektor bereitzustellen.

4. Vorrichtung nach einem der vorhergehenden Ansprüche, **dadurch gekennzeichnet, dass** das Verfahren nach Schritt (230) ein Verfahren zur Verwendung der Vorrichtung bereitstellt, das die folgenden Schritte umfasst:

   (300) Erfassen, in vorgegebenen Zeitintervallen, der von der mindestens einen Elektrode kommenden Signale (Ns) für eine vorgegebene Dauer und sie in einem entsprechenden Puffer Speichern.

(310) Unterteilen jedes der Signale (Ns) in mehrere Zeitintervalle vorgegebener Dauer und so Signalelemente "M" für jedes Signal Erhalten,

(320) Extrahieren, für jedes der in Schritt (310) extrahierten Signalelemente, mindestens eines charakteristischen Parameters (Eigenschaft) gemäß den gleichen Modi wie in Schritt (120),

(330) Berechnen eines statistischen Schätzers des Wertes jedes in Schritt (320) berechneten Merkmals gemäß denselben in Schritt (130) befolgten Modi und einen Vektor Definieren, der durch alle Werte der statistischen Schätzer definiert ist;

(340) Berechnen des euklidischen Abstands zwischen dem in Schritt (330) berechneten Vektor und jedem in Schritt (150) gespeicherten und einer bestimmten Bewegung zugeordneten Vektor und so die Bewegung Identifizieren, für die dieser Abstand minimal ist,

(350) Ausführen der in Schritt (340) identifizierte Bewegung.

5. Vorrichtung nach Anspruch 4, weiterhin umfassend einen Positionssensor, der so konfiguriert ist, dass er mehrere Referenzpositionen erkennt, und **dadurch gekennzeichnet ist, dass** das Verfahren die Wiederholung der Schritte (100) bis (150) für jede der Referenzpositionen durch Speichern für jede von diesen und für jede Bewegung von Schritt (140) sowie für die Ruhebedingung ein Vektor umfasst, der durch alle Werte der in Schritt (130) berechneten Merkmale definiert ist.

6. Vorrichtung nach Anspruch 5, **dadurch gekennzeichnet, dass** das Verfahren vor Schritt (300) außerdem die folgenden Schritte umfasst:

(298) Erfassen, mittels des Positionssensors der Position der prothetischen Vorrichtung und so die signifikante Referenzposition Identifizieren,

(299) Auswählen aller Referenzpositionen, die jede Bewegung in Bezug auf die in Schritt (298) identifizierte Position darstellen.

7. Vorrichtung nach einem der vorhergehenden Ansprüche, **dadurch gekennzeichnet, dass** das Verfahren außerdem ein Verfahren zum automatischen Aktualisieren der in Schritt (150) gespeicherten Vektoren umfasst, das die folgenden Schritte umfasst:

(400) Starten eines Timers mit einer vorgegebenen Zeitspanne, während die Vorrichtung verwendet wird;

(410) Speichern der Werte der Merkmale, die sich auf die erste Bewegung beziehen, nach Ablauf des Timers des Schritts (400), der der Ruhebedingung zugeordnet ist, und so einen ersten Vektor (Rest_1) Definieren, der der ersten Bewegung mit dem Ruhezustand zugeordnet ist, und Starten eines neuen Timers mit einer vorgegebenen Zeitspanne;

(420) Speichern der Werte der Merkmale, die sich auf die erste Bewegung beziehen, nach Ablauf des Timers von Schritt (410), die der Ruhebedingung zugeordnet sind, und so einen zweiten Vektor (Rest _2) Definieren, der der Ruhebedingung zugeordnet ist;

(430) Berechnen der euklidischen Abstände jedes der beiden in den Schritten (410) und (420) definierten Vektoren aus dem Referenzvektor, der die in Schritt (150) gespeicherte Ruhebedingung (Rest_ref) darstellt;

(440) für den Fall, dass der Abstand zwischen dem in Schritt (150) gespeicherten Referenzvektor (Rest_ref) und dem zweiten Vektor, der mit der in Schritt (420) definierten Ruhebedingung verknüpft ist, größer ist als der Abstand zwischen dem gespeicherten Referenzvektor (Rest_ref). in Schritt (150) und dem ersten Vektor, der mit der in Schritt (410) definierten Ruhebedingung verknüpft ist, Aktualisieren der Werte des Referenzvektors entsprechend dem, was in Schritt (410) bekannt ist, und im Gegenteil, diese Werte nicht Aktualisieren.

(450) für den Fall, dass in Schritt (440) eine Aktualisierung der Werte erforderlich ist, indem der Wert jedes i-ten Merkmals des neuen Referenzvektors in Bezug auf die Ruhebedingung (Rext_ref') wie

$$Rest\_ref'(i) = Rest\_ref(i) + b. [Rest\_2(i)] - Rest\_ref(i)] \text{ Brechnen}$$

wobei b ein Koeffizient mit einem Wert zwischen 0 und 1 ist,

(460) Ersetzen der Werte der Merkmale, die sich auf die erste Bewegung beziehen, die mit dem Ruhezustand verbunden ist, der in Schritt (410) angegeben wurde, durch die Werte der zweiten Bewegung, die mit dem Ruhezustand verbunden sind, der in Punkt (420) angegeben wurde, und erneut ab Schritt (420).

8. Vorrichtung nach einem der vorhergehenden Ansprüche, umfassend mindestens einen Aktuator, der mit einer proportionalen Steuerung des Drehmoments und/oder der Geschwindigkeit ausgestattet ist, die von der Prothese oder der Orthese oder dem Exoskelett betätigt wird, und **dadurch gekennzeichnet ist, dass** das Verfahren die Schritte umfasst:

(1000) Definieren der mathematische Form einer Abhängigkeitsfunktion des Ausgangswerts der Proportionalsteuerung als Funktion der für das von jedem Sensor aufgezeichneten Signal berechneten Eigenschaften, wobei die mathematische Form die Summation mindestens eines Termes umfasst, der direkt proportional zu jeder i-ten Charakteristik mittels eines jeweiligen Koeffizienten ($\omega$li) ist,

(1100) Definieren mit einem Wert gleich 1 der Werte aller Koeffizienten ($\omega$ei, $\omega$ni), die die mathematische Form definieren und die sich nicht auf die direkte Proportionalität zu einem Merkmal beziehen;

(1200) Schätzen der Werte der in Schritt (1000) definierten direkten Proportionalitätskoeffizienten ($\omega$1i) mithilfe eines Fehlerreduzierungsalgorithmus, der für eine Vielzahl von prozentualen Werten der für den Aktuator zu verwendenden Anweisung setzt voraus, dass die Summe der während der Ausführung der Bewegung für jedes Merkmal (Fi_max) aufgezeichneten Maxima, jeweils multipliziert mit dem jeweiligen Proportionalitätskoeffizi-enten ($\omega$li) und dem berücksichtigten Prozentwert, gleich den Prozentwerten der Anweisung ist, die für den Aktuator verwendet werden muss.

(1300) Veranlassen des Benutzers, die einer Kalibrierung unterliegende Bewegung zu wiederholen, wobei ihn aufgefordert wird, das angelegte Drehmoment schrittweise zu erhöhen, und ihm über eine grafische Schnittstelle eine Rückmeldung über das angelegte Drehmoment Geben, wobei das angelegte Drehmoment mit Hilfe der definierten Funktion im Schritt (100) berechnet wird, mit den in Punkt (1200) geschätzten Werten der Koeffizienten berechnet wird, die im Punkt (1200) geschätzt werden.

## Revendications

1. Dispositif prothétique comprenant:

   - un membre ou articulation artificielle ou orthèse ou un exosquelette comprenant un mécanisme muni d'au moins un actionneur et configuré pour réaliser une ou plusieurs actions;
   - une source d'alimentation électrique;
   - au moins une source d'entrée modulée par la contraction d'un ou plusieurs muscles du sujet portant le dispositif, comprenant au moins une électrode;
   - des moyens électroniques de calcul, sur lesquels sont chargés des programmes informatiques, configurés pour détecter les signaux issus de ladite au moins une source d'entrée et pour actionner ledit au moins un actionneur afin que ledit mécanisme réalise une action en fonction des signaux issus de ladite au moins une source d'entrée,

   **caractérisé en ce que**
   sur lesdits moyens informatiques sont chargés des programmes informatiques configurés pour mettre en œuvre le procédé comprenant les étapes suivantes:

   (100) enregistrer les signaux (Ns) détectés par ladite au moins une électrode pendant que ledit sujet portant le dispositif est dans une condition prédéterminée de mouvement ou de repos, pendant un intervalle de temps prédéterminé $\Delta$T;

   (110) subdiviser chacun desdits signaux (Ns) en une pluralité d'intervalles de temps de durée prédéterminée;

   (120) extraire du signal relatif à chacun des intervalles de temps définis à l'étape (120) au moins un paramètre caractéristique (propriété) relatif au signal sélectionné dans la liste comprenant: l'amplitude moyenne crête ou crête à crête, la crête ou amplitude maximale crête à crête, valeur efficace, fréquence moyenne du spectre de puissance, fréquence médiane du spectre de puissance, bande spectrale de puissance de -6 dB, bande spectrale de puissance de -20 dB;

   (130) calculer un estimateur statistique pour chaque caractéristique calculée à l'étape (120),

   (140) répéter les étapes (100) à (130) séquentiellement avec ledit sujet au repos et, dans la suite, avec ledit sujet effectuant un mouvement connu sélectionné parmi une liste d'une pluralité de mouvements prédéterminés (Mov_1, Mov_2,..., Move_n) pour chaque itération, (150) associer un vecteur défini par l'ensemble des valeurs des caractéristiques calculées à l'étape (130) à chaque mouvement connu effectué par le sujet et à la condition de repos de l'étape (140), et stocker lesdits vecteurs.

2. Dispositif selon la revendication 1, **caractérisé en ce que** ledit procédé comprend également une procédure de test, comprenant les étapes de:

(200) détecter les signaux détectés par ladite au moins une électrode pendant que l'utilisateur effectue un mouvement spécifique,

(210) calculer, avec les mêmes modes décrits aux étapes (110) à (130), une pluralité d'estimateurs statistiques, et définir un vecteur défini par l'ensemble des valeurs desdits estimateurs statistiques;

(220) individualiser, parmi tous les vecteurs stockés à l'étape (150) et associés à un mouvement différent, celui ayant la distance euclidienne la plus basse du point défini à l'étape (210);

(230) fournir à l'utilisateur un retour indiquant le mouvement individualisé à l'étape (220).

3. Dispositif selon la revendication 2, **caractérisé en ce que** ledit procédé prévoit à l'étape (230) de fournir également à l'utilisateur une indication relative à la distance euclidienne entre le vecteur calculé à l'étape (210) et le vecteur individué à l'étape (220).

4. Dispositif selon l'une quelconque des revendications précédentes, **caractérisé en ce que** ledit procédé prévoit, après l'étape (230), une procédure d'utilisation du dit dispositif, comprenant les étapes de:

(300) acquérir, à intervalles de temps prédéterminés, les signaux (Ns) issus de ladite au moins une électrode, pendant une durée prédéterminée et les stocker dans un tampon relatif,

(310) subdiviser chacun desdits signaux (Ns) en une pluralité d'intervalles de temps de durée prédéterminée, obtenant ainsi des éléments de signal « M » pour chaque signal,

(320) pour chacun des éléments de signal extraits à l'étape (310), extraire au moins un paramètre caractéristique (propriété) selon les mêmes modes suivis à l'étape (120),

(330) calculer un estimateur statistique de la valeur de chaque caractéristique calculée à l'étape (320) selon les mêmes modes suivis à l'étape (130) et définir un vecteur défini par l'ensemble des valeurs desdits estimateurs statistiques;

(340) calculer la distance euclidienne entre le vecteur calculé à l'étape (330) et chaque vecteur stocké à l'étape (150) et associé à un mouvement spécifique, identifiant ainsi le mouvement pour lequel cette distance est minimale,

(350) effectuer le mouvement identifié à l'étape (340) .

5. Dispositif selon la revendication 4, comprenant en outre un capteur de position configuré pour reconnaître une pluralité de positions de référence et **caractérisé en ce que** ledit procédé prévoit de répéter les étapes (100) à (150) pour chacune desdites positions de référence, en mémorisant pour chacun et pour chaque mouvement de l'étape (140) ainsi que pour la condition de repos un vecteur défini par l'ensemble des valeurs des caractéristiques calculées à l'étape (130) .

6. Dispositif selon la revendication 5, **caractérisé en ce que** ledit procédé comprend également, avant l'étape (300), les étapes de:

(298) acquérir au moyen dudit capteur de position la position du dispositif prothétique, identifiant ainsi la position de référence significative,

(299) sélectionner l'ensemble des positions de référence représentant chaque mouvement relatif à la position identifiée dans l'étape (298).

7. Dispositif selon l'une quelconque des revendications précédentes, **caractérisé en ce que** ledit procédé comprend également une procédure de mise à jour automatique des vecteurs stockés à l'étape (150), comprenant les étapes de:

(400) pendant que le dispositif est utilisé, démarrer un temporisateur d'une période prédéterminée;

(410) stocker les valeurs des caractéristiques relatives au premier mouvement après l'expiration du temporisateur de l'étape (400) associée à la condition de repos, définissant ainsi un premier vecteur (Rest_1) associé audit premier mouvement associé à la condition de repos, et démarrant une nouvelle minuterie de période prédéterminée ;

(420) stocker les valeurs des caractéristiques relatives au premier mouvement après l'expiration du temporisateur de l'étape (410) associée à la condition de repos, définissant ainsi un deuxième vecteur (Rest_2) associé à la condition de repos;

(430) calculer les distances euclidiennes de chacun des deux vecteurs définis aux étapes (410) et (420) à partir du vecteur de référence représentant la condition de repos (Rest_ref) stocké à l'étape (150);

(440) dans le cas où la distance entre le vecteur de référence (Rest_ref) stocké à l'étape (150) et ledit deuxième vecteur associé à la condition de repos définie à l'étape (420) est supérieure à la distance entre le vecteur de référence (Rest_ref) stocké à l'étape (150) et ledit premier vecteur associé à la condition de repos définie à l'étape (410), mettant à jour les valeurs dudit vecteur de référence selon ce qui est connu à l'étape (450), au contraire, ne mettant pas à jour lesdites valeurs.

(450) dans le cas où à l'étape (440) il est nécessaire de mettre à jour les valeurs, en calculant la valeur de chaque caractéristique i-ème du nouveau vecteur de référence par rapport à la condition de repos (Rext_ref') comme

$$Rest\_ref'(i) = Rest\_ref(i) + b. \, [Rest\_2(i) - Rest\_ref(i)]]$$

dans laquelle b est un coefficient de valeur comprise entre 0 et 1,

(460) remplacer les valeurs des caractéristiques relatives au premier mouvement associé à la condition de repos individuée à l'étape (410) par les valeurs du deuxième mouvement associé à la condition de repos individuée au point (420), et de nouveau à partir de l'étape (420).

8. Dispositif selon l'une quelconque des revendications précédentes, comprenant au moins un actionneur doté d'un contrôle proportionnel du couple et/ou de la vitesse actionné par la prothèse ou l'orthèse ou l'exosquelette, et **caractérisé en ce que** ledit procédé comprend les étapes de:

(1000) définir la forme mathématique d'une fonction de dépendance de la valeur de sortie de ladite commande proportionnelle en fonction des caractéristiques calculées pour le signal enregistré par chaque capteur, ladite forme mathématique comprenant la sommation d'au moins un terme directement proportionnel à chaque ième caractéristique au moyen d'un coefficient respectif ($\omega li$)

(1100) définir avec une valeur égale à 1 les valeurs de tous les coefficients ($\omega ei$, $\omega ni$) définissant ladite forme mathématique et qui ne sont pas relatifs à la proportionnalité directe à une caractéristique ;

(1200) estimer les valeurs desdits coefficients de proportionnalité directe ($\omega 1i$) définis à l'étape (1000) au moyen d'un algorithme de réduction d'erreur qui, pour une pluralité de valeurs en pourcentage de l'instruction à utiliser pour l'actionneur, impose que la sommation des maximums enregistrés lors de l'exécution du mouvement pour chaque caractéristique ($Fi\_max$), multipliée chacun par le coefficient de proportionnalité respectif ($\omega li$) et par la valeur en pourcentage considérée, est égal aux valeurs en pourcentage de l'instruction à utiliser pour l'actionneur.

(1300) faire répéter à l'utilisateur le mouvement objet de l'étalonnage, en lui demandant d'augmenter progressivement le couple appliqué, et lui fournir un retour au moyen d'une interface graphique du couple appliqué, ledit couple appliqué étant calculé au moyen de ladite fonction définie à l'étape (100), avec les valeurs des coefficients estimées au point (1200) .

**EP 4 221 641 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016278947 A
- US 10448857 B2
- US 9566016 B2

**Non-patent literature cited in the description**

- **MERLETTI.** Electromyography: Physiology, Engineering, and Non-Invasive Applications. *IEEE Press Series on Biomedical Engineering,* 2004